# EUROPEAN PATENT APPLICATION

(11) **EP 2 042 604 A1**
(43) Date of publication of application: **01.04.2009**
(21) Application number: 07117296.9
(22) Date of filing: 26.09.2007
(51) Int. Cl.: C12N 15/863, A61K 39/00, A61K 39/12

(54) **VV promoter driven overexpression of recombinant antigens**

(71) Applicant: Helmholtz Zentrum München Deutsches Forschungszentrum für Gesundheit und Umwelt (GmbH), 85764 Neuherberg (DE); Helmholtz-Zentrum für Infektionsforschung GmbH, 38124 Braunschweig (DE)
(72) Inventor: Drexler, Ingo, 80797, München (DE); Kastenmueller, Wolfgang, 81671, München (DE); Gasteiger, Georg, 81675, München (DE); Guzmann, Carlos, 38304, Wolfenbuettel (DE); Becker, Pablo Daniel, 38300, Wolfenbuettel (DE); Noerder, Miriam, 27753, Delmenhorst (DE)
(74) Representative: Sandmann, Wolfgang

(57) **Abstract**

The present invention is directed to a pharmaceutical composition comprising a vaccinia virus (VV) based vector construct under the control of a strong VV specific promoter. The present invention is further directed to the use of this pharmaceutical composition for the treatment of infectious diseases, chronic inflammatory or degenerative diseases, autoimmunity diseases, fungal diseases, cancer and allergies and for evoking an immune response against subdominant and/or cryptic epitopes.

## Description

The present invention is directed to a pharmaceutical composition comprising a vaccinia virus (VV) based vector construct under the control of a strong VV specific promoter. The present invention is further directed to the use of this pharmaceutical composition for the treatment of infectious diseases, chronic inflammatory or degenerative diseases, autoimmunity diseases, fungal diseases, cancer and allergies and for evoking an immune response against subdominant and/or cryptic epitopes.

### Background of the invention

Recombinant vaccinia viruses (VV) have become attractive vectors for antigen delivery in basic and clinical research. In particular, the VV strain MVA is a highly immunogenic vector vaccine and able to efficiently induce primary and to amplify secondary immune responses.

In 1953, German scientists obtained the vaccinia virus Ankara strain, which was originally obtained at the Turkish institute in Ankara by passages alternating between donkeys and calves (Mayr, A., Hochstein-Mintzel, V. and Stickl, H. (1975) Passage history, properties, and applicability of the attenuated vaccinia virus strain MVA (in German). Infection 3, 6-14.). The virus strain was established as chorioallantois vaccinia virus Ankara (CVA), because it was routinely cultivated and titered on the chorioallantois membrane of embryonated chicken eggs. Some years later, A. Mayr reported that CVA had developed attenuated growth characteristics on the chorioallantois membrane, in tissue cultures and in laboratory animals after 371 passages on chicken embryo fibroblasts (CEFs) in the Bavarian State Vaccine Institute. Upon further passages the phenotype was stabilised, and the 516^{th} passage virus was renamed as modified vaccinia virus Ankara. As consequence of these passages the virus was noted to be less virulent than its parent strain. From 1968, MVA vaccine has been safely administered to more than 100,000 people in Germany, including many persons considered at risk for conventional vaccination (e.g., immunocompromised, elderly) without documentation of any complication. Today, MVA vectors are handled in USA and Germany under biosafety level 1 conditions.

Recently, the complete genome of MVA has been sequenced (from an MVA preparation provided as standard obtained in the 572^{nd} CEF passage), and the genes lost during attenuation have been identified (Antoine, G., Scheiflinger, F., Domer, F. and Falkner, F.G. (1998) The complete genomic sequence of the modified vaccinia Ankara strain: comparison with other orthopoxviruses. Virology 244, 365-96). During attenuation, MVA lost ∼15% of its CVA parent genome (ca. 30 kb) including genes for virus host range regulation and evasion of the host immune response. Thus, MVA replication is extremely limited in mammalian cells, being blocked at a very late stage of the virus life cycle with no alteration in early or late virus gene expression or in the levels of expression of heterologous proteins. It was demonstrated that the immune responses stimulated against a foreign MVA-encoded antigen were at least as high, or even higher at certain viral doses, than those stimulated by a replication competent strain. Early events in the life cycle and/or advantages in transcription or translation due to multiple deletions in functional genes could account for this enhancement. The more efficient foreign gene expression is cell type independent.

Furthermore, several natural deletion sites can be found in the genome of MVA distributed in clusters, which can serve as insertion sites for foreign genes without affecting MVA gene functions. The deletions II, III and VI have been preferentially used because they are located in the more conserved regions of the genome, therefore, they are not affected by sequence rearrangements. The viral thymidine kinase is the standard insertion locus used in recombinant vaccinia viruses and it has been also used to obtain recombinant MVA. The integration of a foreign gene sequence into the MVA genome can be directed by homologous recombination using transfer plasmids that contain MVA DNA fragments homologous to DNA sequences flanking the targeted insertion site. In addition, the K1L gene is not expressed in MVA, causing an early block of viral replication upon infection of rabbit kidney RK-13 cells. However, when MVA is complemented, the defective virus is able to grow in these cells. Thus, a method to obtain recombinant MVA was developed, in which the transient introduction of the K1L gene into the viral genome can be used as a selectable growth marker.

An important issue to be considered in the context of using MVA as a vector is the role played by pre-existing immunity against vaccinia virus in a large part of the human population (Ramirez, J.C., Gherardi, M.M., Rodriguez, D. and Esteban, M. (2000) Attenuated modified vaccinia virus Ankara can be used as an immunizing agent under conditions of preexisting immunity to the vector. J Virol 74, 7651-5). It has been suggested that, if recombinant poxviruses are applied to humans, these individuals can not receive further vaccines based on the same vectors. However, it was shown that MVA is less affected by pre-existing vaccinia-specific immunity than the replication competent Western Reserve (WR) strain (Ramirez, J.C., Gherardi, M.M., Rodriguez, D. and Esteban, M. (2000). Attenuated modified vaccinia virus Ankara can be used as an immunizing agent under conditions of preexisting immunity to the vector. J Virol 74, 7651-5). Weak anti-virus immune responses were triggered by MVA at both humoral and cellular levels. However, the immune responses against the foreign antigen were nearly fourfold higher than those observed in mice pre-immunised with the WR strain. The potential problem of impaired effectiveness of the MVA vector because of pre-existing immunity can be overcome by different strategies. The use of either different routes for priming and boosting or the combination with DNA vaccines could provide an answer. It was demonstrated that after subcutaneous vaccination with vaccinia, the inductive sites of the mucosal immune system may still be naïve to the vaccinia antigens. Therefore, immunisation by the mucosal route can be used for the induction of recombinant protein-specific responses in individuals with pre-existing immunity to the carrier.

Basically, the usefulness of vaccines in the fight against infections has been clearly demonstrated by the eradication programs against smallpox and polio. Nevertheless, there is considerable number of diseases for which vaccines are not yet available or the existing ones are unsatisfactory. Whole cell vaccines are systematically being replaced by subunit vaccines, in which purified antigens or their coding genes are exploited in combination with new adjuvants and/or delivery systems. However, purified antigens are usually poorly immunogenic, raising the problem of stimulating adequate immune responses following vaccination. Thus, recombinant poxviruses (see above) have been successfully exploited as vaccine vectors.

However, there is little knowledge about the interdependence of immune responses directed against recombinant target or VV, for example, MVA vector antigens. In addition, intrinsic properties of the target antigen which is expressed may negatively influence the immunogenicity, particularly if adaptive immune responses have to compete with those directed against the viral vector backbone. Although MVA is an extremely potent approach to boost immune responses, it is not always possible to promote strong responses using it as a priming system. Thus, there is still a need to improve VV based vector systems to promote optimal immune responses.

Therefore, a problem underlying the present invention is to provide a vaccine, which is capable of inducing an immune response, in particular T cell response, against not only dominant, but also cryptic or subdominant epitopes. It is a further problem underlying the present invention to provide a broad immune reaction against a desired antigen. It is a still further problem of the invention to provide a VV vector based vaccine, which allows an effective immune response against an antigen (epitope) encoded by the VV vector based vaccine, wherein the vaccine contains a considerably lower number (dosage) of VV than state of the art vaccines.

### Summary of the invention

To overcome these problems, the inventors have investigated whether an increased amount of target antigens achieved by overexpression via strong and potent VV promoters may be a tool to enhance the immunogenicity of antigenic determinants delivered by recombinant MVA vaccines. They have performed among others comparative studies using VV promoters with i) moderate activity in the early as well as in the late phase of the viral infection such as the natural VV promoter P7.5 (Prom A), ii) very strong activity in the early phase, but moderate activity in the late phase such as the genetically modified VV promoter PH5 (Prom B) and iii) strong activity in the early phase and very strong activity in the late phase of the viral life cycle like the synthetic promoter sP (Prom C).

The strategy disclosed herein basically relies on a pharmaceutical composition comprising a vaccinia virus (VV) based vector construct, wherein the vector construct encodes a heterologous antigen under the control of a *strong* VV specific promoter; and a pharmaceutically acceptable carrier.

It unexpectedly turned out that this composition brought about several advantages, which are shortly outlined in the following:
1. By using a strong promoter, it is possible to expand the strength and broadness of cellular immune responses. This fact is not only due to the enhanced activity of the strong promoter (in the meaning of enhancing the level of transcription of a given gene leading to overexpression), but led to different transcriptional products, which presumably caused an expanded immune response. Without being bound to a specific theory, it is assumed that the reading mistake rate by using a strong promoter is higher, for example by ignoring a stop signal und, thus, fragments of different length are produced. Or in other words, the stronger the promoter is, the higher the number of unexpected transcriptional products will be. This allows for the evocation of a broad and strong immune response also against subdominant and/or cryptic epitopes.
2. It surprisingly turned out that by using a strong promoter in this context, the overall number of viruses to be administered to a patient can be reduced considerably, thus, leading to a reduced risk of unwanted side effects which are due to the viruses themselves.

### Detailed description of the application

According to a first aspect, the present invention provides a pharmaceutical composition comprising
a) a vaccinia virus (VV) based vector construct, wherein the vector construct encodes a heterologous antigen under the control of a *strong* VV specific promoter; and
b) a pharmaceutically acceptable carrier.

The ingredients of the present invention are used in form of a pharmaceutical composition where they are mixed with suitable carriers or excipients in doses to treat or ameliorate the disease. Such a composition may also contain (in addition to the ingredient and the carrier) diluents, fillers, salts, buffers, stabilizers, solubilizers and other materials well known in the art. The term "pharmaceutically acceptable" means a non-toxic material that does not interfere with the effectiveness of the biological activity of the active ingredient(s). The characteristics of the carrier will depend on the route of administration. The pharmaceutical composition may further contain other agents which enhances the activity of the active ingredient. Such additional factors and/or agents may be included in the pharmaceutical composition to produce a synergistic effect or to minimize side-effects.

Techniques for formulation and administration of the compounds of the instant application may be found in "Remington's Pharmaceutical Sciences", Mack Publishing Co., Easton, PA, latest edition. Whenever the compositions are to be used for medical purposes, they will contain a therapeutically effective dose of the respective ingredient. A therapeutically effective dose further refers to that amount of the compound/ingredient sufficient to result in amelioration of symptoms, e.g., treatment, healing, prevention or amelioration of such conditions.

An epitope as defined herein is the part of a macromolecule that is recognized by the immune system, specifically by antibodies, B cells, or T cells. Although epitopes are usually thought to be derived from non-self proteins, sequences derived from the host that can be recognized are also classified as epitopes.

Most epitopes recognized by antibodies or B cells can be thought of as three-dimensional surface features of an antigen molecule; these features fit precisely and thus bind to antibodies. Exceptions are linear epitopes, which are determined by the amino acid sequence (the primary structure) rather than by the 3D shape (tertiary structure) of a protein.

T cell epitopes are presented on the surface of an antigen-presenting cell, where they are bound to MHC molecules. T cell epitopes presented by MHC class I molecules are typically peptides between 8 and 11 amino acid in lengths, while MHC class II molecules present longer peptides, and non-classical MHC molecules also present non-peptidic epitopes such as glycolipids.

Epitopes can be mapped using protein microarrays, and with the ELISPOT or ELISA techniques.

By using strong promoters of the present invention, antigenic fragments are expressed carrying subdominant and/or cryptic epitopes. These are defined herein as epitopes which are of low or no immunogenicity due to either permanent or temporal low copy number on the cell surface, e.g. because of insufficient or delayed processing, MHC binding or presentation, or suppression of T cells potentially reactive for these epitopes by T cells reactive for other specificities e.g. dominant epitopes. By using the present pharmaceutical composition, it will be possible to evoke also a broad immune response against these epitopes.

In a preferred embodiment, the heterologous antigen is a pathogenic antigen. It is noted that the term "heterologous" as used herein means that the antigen is derived from a different species.

Preferably, the pathogenic antigen is derived from the group consisting of viruses, bacteria, protozoa, fungi and parasites as well as tumor cells or tumor cell associated antigens and functional parts thereof. Further examples of such pathogenic antigens are described in Davis, B.D. et al., (Microbiology, 3rd ed., Harper International Edition).

For example, the viruses are selected from the group consisting of influenza viruses, measles and respiratory syncytial viruses, dengue viruses, human immunodeficiency viruses, human hepatitis viruses, herpes viruses, or papilloma viruses.

In a preferred embodiment, the construct encodes an antigen selected from carcinoembryonic antigen (CEA), Her-2/neu, Gag of HIV and PA derived from the polymerase complex of Influenza.

In a specifically preferred embodiment, the VV is modified vaccinia virus Ankara (MVA). The origin and nature of this vector has been explained in detail in chapter "Background of the Invention", to which it is fully referred herewith.

The term "strong promoter" as used herein means that compared to naturally occurring promoters such as the Prom A promoter with a defined moderate activity in the early and late phase, a strong promoter has an enhanced activity of gene expression either in the early or late phase of infection or both resulting in higher amounts of antigen produced.

Preferably, the promoter is a promoter having strong activity in the early and/or late phase of the viral life cycle. As a particularly preferred promoter, Prom B or Prom C should be mentioned. As indicated above, Prom B is a genetically modified VV promoter and shows a very strong activity in the early phase, but moderate activity in the late phase. Prom C is a synthetic promoter showing a strong activity in the early phase and a very strong activity in the late phase of the viral life cycle.

In a preferred embodiment, the Prom B promoter is in accordance with SEQ ID NO:1, and/or the Prom C promoter is in accordance with SEQ ID NO:2.
SEQ ID NO:1: Sequence of the modified H5 promoter PH5 (Prom B) 
TAATCGTGTCATATTAGTATAAAAAGTGATTTATTTTACAAAATTATGTATTTTG
TTCTATCAACTACCTATAAAACTTTCCCTGCAGGTCAGCTTAAAAATTGAAATTTT
ATTTTTTTTTTTGGAATATAAATAA
SEQ ID NO:2: Sequence of the synthetic promoter sP (Prom C)

TATTGGTAATCGTGTCATATTAGTATAAAAAGTGATTTATTTTTACAAAATTATGT
ATTTGTTCTATCAACTACCTATAAAACTTTCCCTGCAGGTCAGCTTAAAAATTGA
AATTTTATTTTTTTTTTTGGAATATAAAT

Further examples of strong promoters are:
SEQ ID NO:3: Sequence of the natural early promoter PK1L 
GGGGGTCTATTAGAGATATTATTAATTCTGGTGCAATATGACAAAATTATAAAAAA
TGAAAAAATATACACTAATTAGCGTCTC
SEQ ID NO:4: Sequence of the natural late promoter P11

CAATCACCACTTTCATATTTAGAATATATGTATGTAAAAAATATAGGAGAATTTCA
TGGGGGGTTTTTCTATGCTATAAATT

In an embodiment, the VV is MVA and the site of insertion of the sequence encoding the heterologous antigen is a non essential site in the viral genome (i.e. is integrated at a site in the viral DNA which is non-essential for the life cycle of the virus), preferably into deletion III. Furthermore, several other natural deletion sites can be found in the genome of MVA distributed in clusters, which can serve as insertion sites for foreign genes without affecting MVA gene functions. The deletions II and VI have been also used because they are located in the more conserved regions of the genome, therefore, they are not affected by sequence rearrangements.

In a second aspect, the invention is directed to a combined composition comprising the following ingredients:
a) a first composition according to one or more of the preceding claims, provided for priming;
b) a second composition according to one or more of the preceding claims, provided for boosting.

The dosages of the two compositions preferably may be chosen as follows:
1 x 10⁷ IU to 5 x 10⁸ IU for priming and 5 x 10⁷ IU to 1 x 10⁹ IU for boosting.

In a preferred embodiment, the pharmaceutical composition is a vaccine.

To prepare vaccines, the VV (MVA) viral vectors according to the invention are converted into a physiologically acceptable form. This can be done based on the many years of experience in the preparation of vaccines used for vaccination against smallpox (Kaplan, Br. Med. Bull. 25, 131-135 [1969]). Typically, about 10⁶-10⁷ for VV and 10⁷-10⁹ for MVA viral particles are freeze-dried in 100ml of phosphate-buffered saline (PBS) in the presence of 2% peptone and 1% human albumin in an ampoule, preferably a glass ampoule. The lyophilisate can contain extenders (such as mannitol, dextran, sugar, glycine, lactose or polyvinylpyrrolidone) or other aids (such as antioxidants, stabilizers, etc.) suitable for parenteral administration. The glass ampoule is then sealed and can be stored, preferably at temperatures below -20°C, for several months.

For vaccination the lyophilisate can be dissolved in 0.1 to 0.2 ml of aqueous solution, preferably physiological saline, and administered parenterally, for example by intradermal inoculation. The vaccine according to the invention is preferably injected intracutaneously. Slight swelling and redness, sometimes also itching, may be found at the injection site. The mode of administration, the dose and the number of administrations can be optimized by those skilled in the art in a known manner. It is expedient where appropriate to administer the vaccine several times over a lengthy period in order to obtain a high level immune responses against the foreign antigen.

The physician in any event will determine the actual dosage which will be most suitable for an individual patient and will vary with the age, weight and response of the particular patient. There can, of course, be individual instances where higher or lower dosage ranges are merited, and such are within the scope of this invention.

In a third aspect, the present invention is directed to the use of the pharmaceutical composition as defined above in the manufacture of a medicament for the treatment of infectious diseases, chronic inflammatory or degenerative diseases, autoimmunity diseases, fungal diseases, cancer and allergies, or, basically in evoking an immune response against subdominant and/or cryptic epitopes.
In a further aspect, a method of evoking an immune response in a patient against an antigen is disclosed comprising subdominant or cryptic epitopes, comprising the steps of:
a) providing a pharmaceutical composition as defined herein,
b) administering said composition to said patient in a suitable amount, thereby evoking an immune response in said patient.

The immune response preferably is a cellular immune response.

The patient subject to this treatment preferably is a mammalian, preferably a human patient. The method of the present invention preferably is performed as a prime boost-regimen.

The present invention is further illustrated by the enclosed figures and examples.

The figures are showing the following:

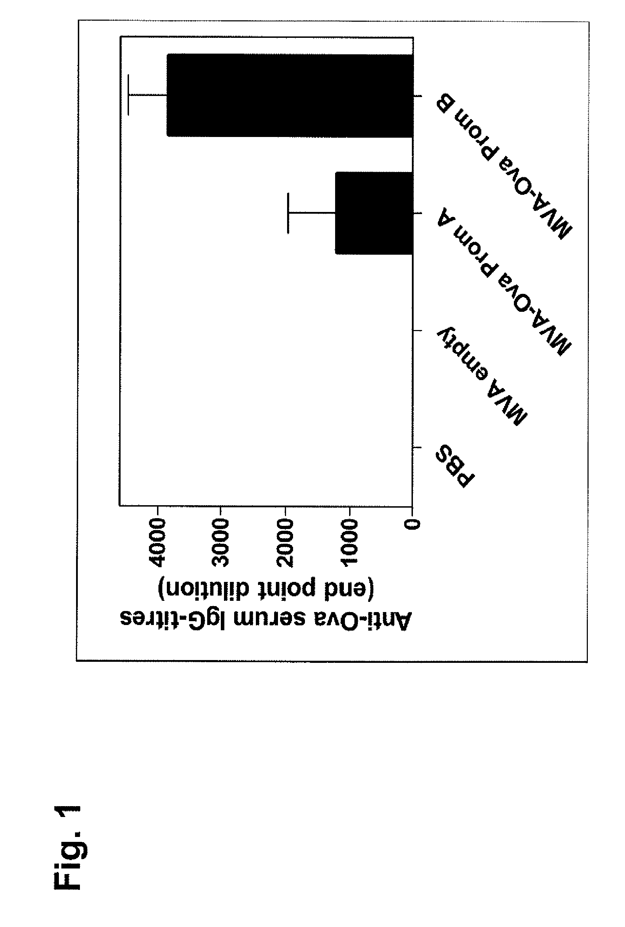

**FIGURE 1****.** Production of anti-Ova serum IgG in mice vaccinated using two different recombinant MVAs. Each bar represents the group mean end-point titre of anti-Ova IgG. The standard error of the mean is indicated by vertical lines.

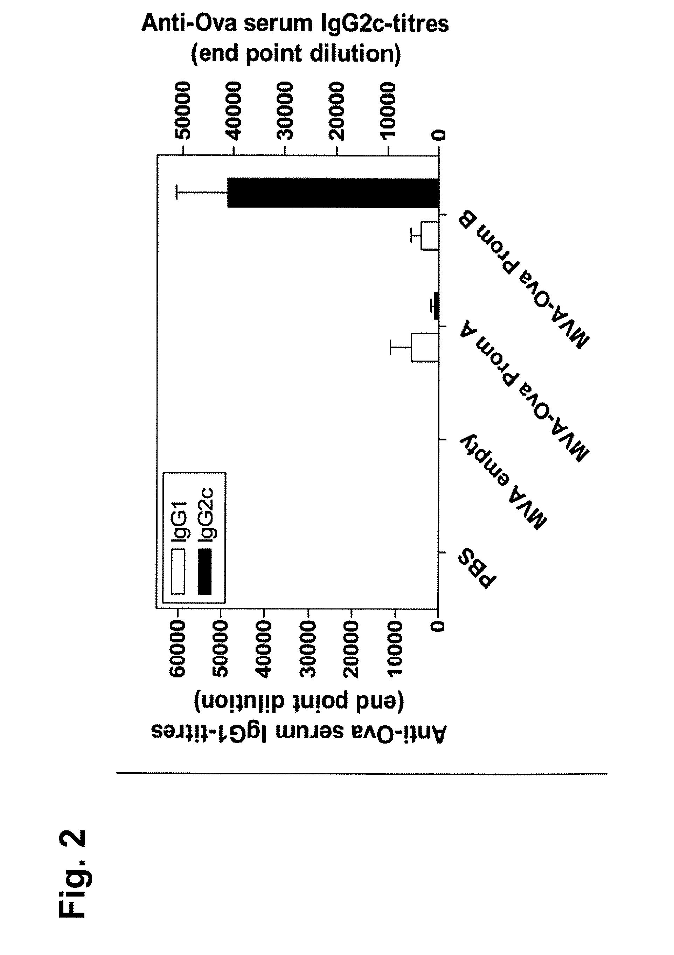

**FIGURE 2****.** Anti-Ova serum IgGI/IgG2c-titres obtained in mice vaccinated using different recombinant MVAs. Each bar represents the group mean end-point titre of anti-Ova IgG. The standard error of the mean is indicated by vertical lines.

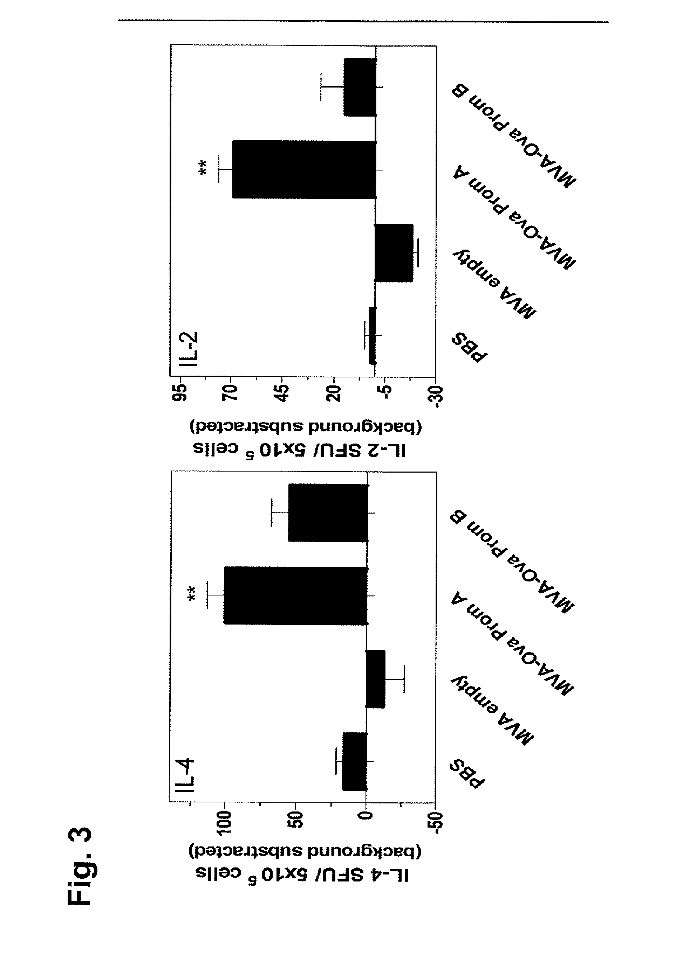

**FIGURE 3****.** Numbers of IL-2- and IL-4-producing cells in mice vaccinated using two different recombinant MVAs. Cells were re-stimulated with Ova protein. Results are presented as specific IL-4- (left panel) and IL-2-spot forming units (SFU)/5 x 105 cells (right panel). The standard error of the mean of quadruplicate values is indicated by vertical lines. The values reported are those obtained from stimulated cells subtracted of background from unstimulated cells. Unstimulated cells were lower than 30 SFU/5 x 105 cells. The results were statistically significant when compared with ANOVA at p<0.01 (**).

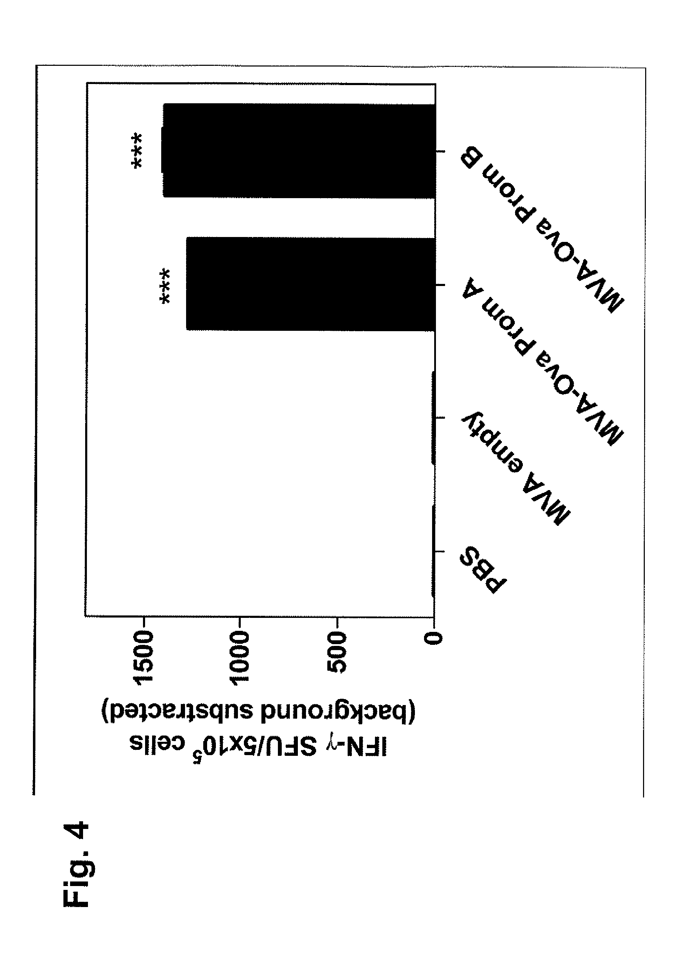

**FIGURE 4**. Numbers of IFN-γ secreting cells. (A) Cells were re-stimulated with the dominant Ova-peptide SIINFEKL. Results are presented as specific spot forming units (SFU)/5x105. The standard error of the mean of quadruplicate values is indicated by vertical lines. The values shown were the results of those obtained from stimulated cells subtracted of background from non-stimulated cells. Plate was analysed with ImmunoSpot Series 3A Analyzer. Unstimulated cells showed a low number of IFN-γ secreting cells (below 30). (*** p<0.001)

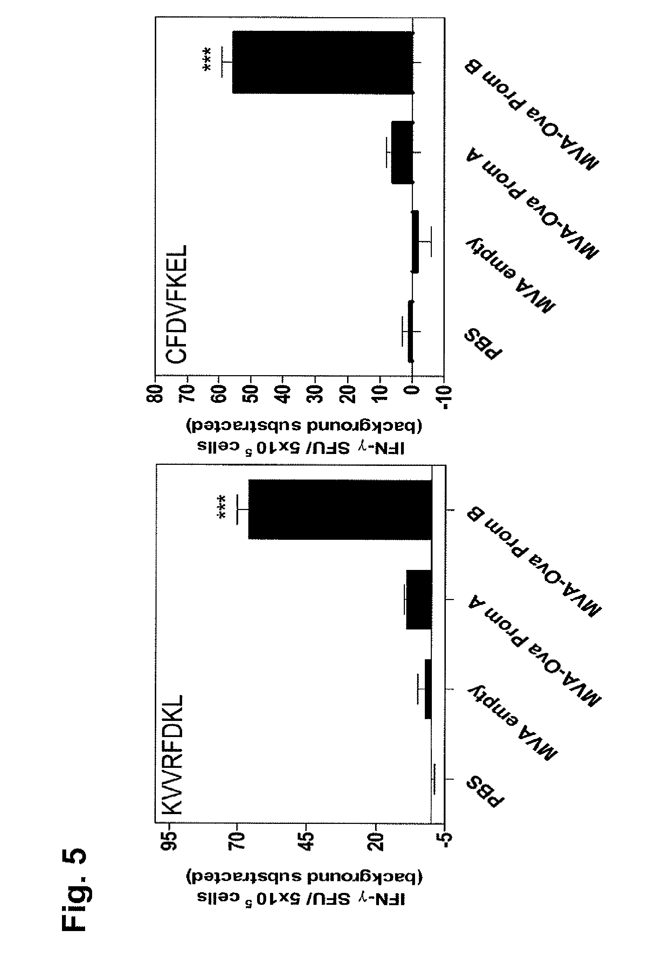

**FIGURE 5****.** Numbers of IFN-γ secreting cells. Cells were re-stimulated with the subdominant (left panel) and cryptic (right panel) Ova-peptides. Results are presented as specific IFN-γ spot forming units (SFU)/5x105. The standard error of the mean of quadruplicate values is indicated by vertical lines. The values shown were the results of those obtained from stimulated cells subtracted of background from non-stimulated cells. Unstimulated cells showed a low number of IFN-γ secreting cells (below 30). (*** p<0.001)

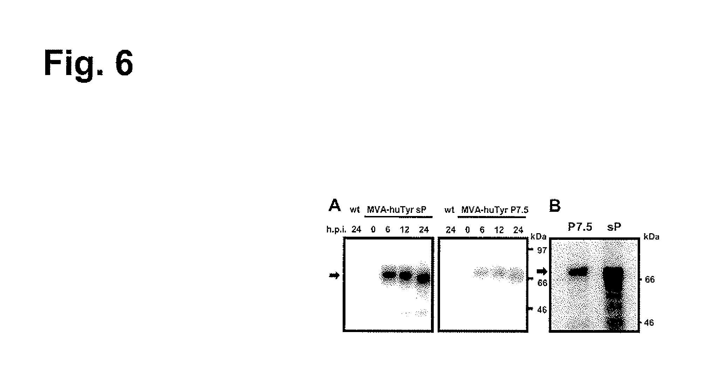

**FIGURE 6****.** Expression of the human *tyrosinase* gene by recombinant MVA under control of a moderate (Prom A, also termed P7.5) or strong promoter (sP) in human HeLa cells. Radioimmunoblot analysis of HeLa cells (A) infected with MVA-hTyr P7.5, MVA-hTyr sP or MVA-wt (lane wt). Cells were harvested at the indicated hours post infection (h.p.i.). Cell lysates were separated by 8% SDS-PAGE. The blot from this gel was probed with anti-Tyr mAb T311 and ¹²⁵I-labeled sheep anti-mouse IgG secondary antibody, and visualized on a phosphorimager. Infection with MVA-wt (lane wt) served as a negative control. The numbers on the right indicate the positions and molecular masses (in kDa) of protein standards. The protein band corresponding in size to glycoforms of tyrosinase is marked by an *arrowhead*. Radioimmunoblot analysis of early gene expression in HeLa cells (B) infected with MVA-hTyr P7.5 or MVA-hTyr sP in the presence of cytosine β-D-arabinofuranoside (AraC). Cells were harvested at 24 h.p.i. and cell lysates prepared as described above.

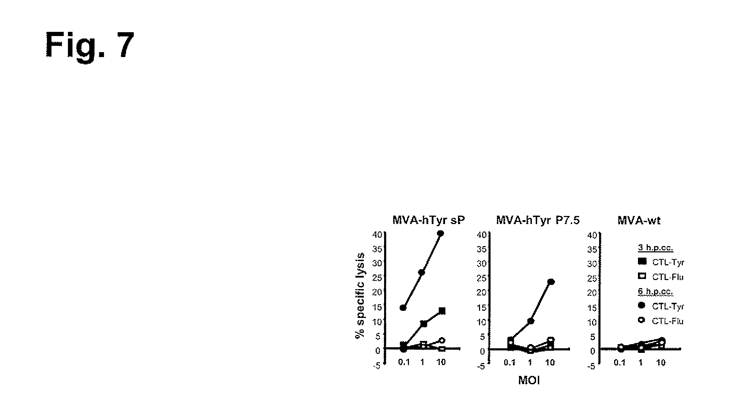

**FIGURE 7****.** Antigenicity of infected target cells in a time- and dose-dependent manner. Cells were infected with recombinant MVA expressing human Tyr under control of a natural moderate (Prom A (P7.5)) or synthetic strong promoter (Prom C (sP)). Specific lysis by A*0201-restricted murine CTL specific for either the human Tyr peptide epitope 369-377 (CTL-Tyr) or the influenza M1 58-66 peptide epitope (CTL-Flu) used as effector cells at an E/T ratio of 30:1 in a 3-h (squares) or 6-h (circles) [⁵¹Cr]-release assay. [⁵¹Cr]-labeled A375 cells were infected with increasing infectious units (IU) of either MVA-hTyr P7.5, MVA-hTyr sP or MVA-wt as indicated. Effector cells including CTL specific for the influenza M1 58-66 peptide epitope (CTL-Flu) which served as a control were used at an E/T ratio of 45:1. Supernatants of effector-target cell cultures were harvested at the indicated hours post co-culture (h.p.c.c.) corresponding to 1-h to 6-h [⁵¹Cr]-release assays.

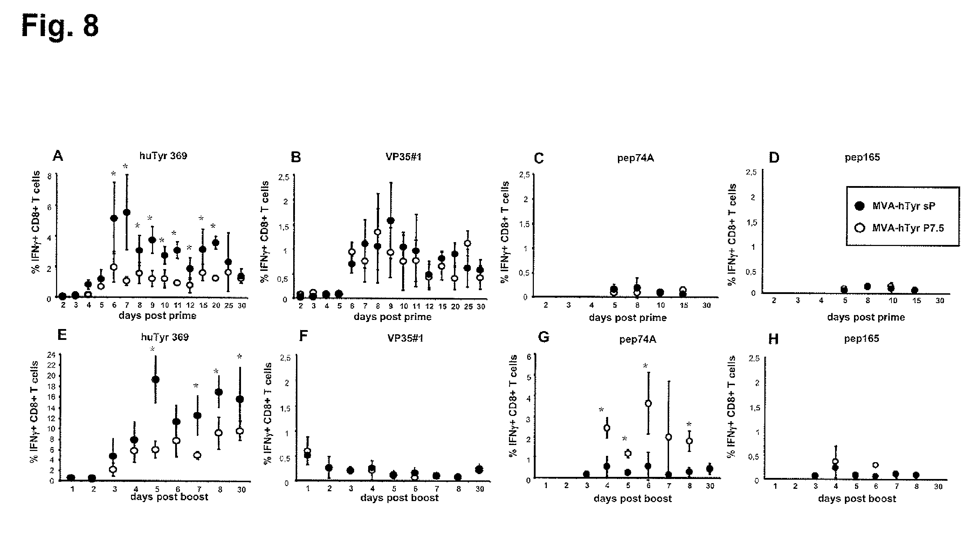

**FIGURE 8****.** Prime-boost kinetics of HLA-A*0201-restricted Tyr and VV epitope-specific CD8+ T cell responses *in vivo*. Quantification of epitope-specific T cells was performed following one (A, C, E) or two immunizations (B, D, F) of HHD-mice with either MVA-hTyr sP or MVA-hTyr P7.5. For booster immunizations, mice were vaccinated a second time 30 days following the first injection. Splenocytes were harvested at the indicated days post prime (A, C, E) or post boost (B, D, F) and stimulated with the human Tyr epitope huTyr 369 (A, B), the VV epitopes VP35#1 (C, D) and pep74A (E, F) or the influenza M1 58-66 peptide as a control. Cells were analyzed by flow cytometry for the presence of peptide-specific, activated (CD62L^{low}) CD8+ T cells. The magnitude of the induced T cell response is depicted as percentages of cytokine secreting CD8+ T-cells within the live (EMA^{negative}) and CD8^{positive} cell population. All results are expressed as mean ± SD for four to six mice. As indicated by an asterisk (*), there is a statistically significant difference between MVA-hTyr sP and MVA-hTyr P7.5 ranging between p< 0.05 to p < 0.0005 (two-tailed, unpaired *t* test).

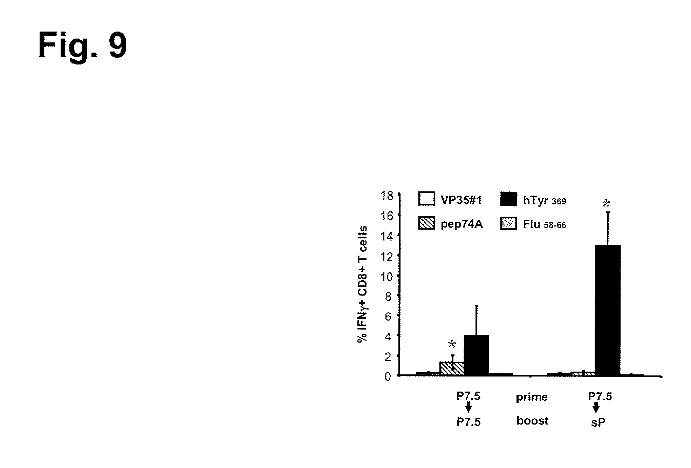

**FIGURE 9****.** Acute phase Tyr- and VV-specific CD8+ T cell responses induced by heterologous prime-boost immunizations with recombinant MVA viruses. All mice were primed i.p. with 10⁷ IU of MVA-hTyr P7.5. MVA- and Tyr-specific T-cell responses were analyzed on day 5 after a second i.p. immunization with 10⁷ IU of either MVA-hTyr P7.5 (P7.5) or MVA-hTyr sP (Prom C (sP)). Splenocytes were stimulated with the human Tyr peptide hTyr 369 (black bar), the VV peptides VP35#1 (white bar) and pep74A (striped bar) or the influenza M1 58-66 peptide (grey bar) as a control. Cells were analyzed by flow cytometry for the presence of peptide-specific, activated (CD62L^{low}) CD8+ T cells. The magnitude of the induced T cell response is depicted as percentages of cytokine secreting CD8+ T-cells within the live (EMA^{negative}) and CD8^{positive} cell population. All results are expressed as mean ± s.d. for four mice. As indicated by an asterisk (*), there is a statistically significant difference between MVA-hTyr sP and MVA-hTyr P7.5 concerning pep74A (p< 0.04) and huTyr 369 (p < 0.007) (two-tailed, unpaired *t* test).

### Examples:

MVA vectors expressing target antigens under control of Prom B or Prom C producing in higher amounts of antigenic determinants in either the early (Prom B) or the late phase of infection (Prom C) as compared to Prom A resulted in stronger immune responses compared to those obtained with MVA vaccines encoding the target antigens under control of Prom A.

The use of MVA vaccines expressing ovalbumin (Ova) under control of Prom B resulted in stronger immune responses on the humoral (Fig. 1) and cellular level (Fig. 4 and 5). These showed a dominant T_{H}1 response pattern characterized by a high number of IFN γ - secreting CD8+ T cells (Fig. 4 and 5) and predominant Ova-specific IgG2c antibody responses (Fig. 2). In contrast, Prom A induced humoral and cellular immune responses were of a T_{H}2 response pattern demonstrating elevated Ova-specific IgG1 antibody titers (Fig. 2) and IL-2 and IL-4 secreting Ova-specific CD8+ T cell responses (Fig. 3). Importantly, this strategy allowed the stimulation of immune responses not only against dominant (Fig. 4), but also against subdominant and cryptic epitopes (Fig. 5). Eliciting a much broader repertoire of T cell specificities argues for a potentially higher performance of this viral vector construct under field conditions.

Additionally, we demonstrate that distinct quantities of target antigen delivered by vectors based on MVA have considerable impact on the magnitude and quality of recombinant antigen and MVA vector-specific CD8+ CTL responses *in vitro* and *in vivo. In vitro*, overexpression of tyrosinase (Tyr) (Fig. 6) resulted in a more vigorous Tyr-specific CTL activity being detectable at earlier times of infection and requiring lower doses of virus (Fig.7). *In vivo*, we performed a comparative monitoring of recombinant antigen and MVA epitope-specific CD8+ T cell responses in the acute and memory phase after vaccination of HLA-A*0201-transgenic HHD mice. Enhanced Tyr synthesis by Prom C-containing MVA constructs induced Tyr-specific CD8+ T cells i) more rapidly and ii) at higher frequency in acute phases, and iii) maintained more memory CTL after secondary vaccinations (Fig. 8 and 9). Moreover, the pattern of immunodominance for MVA epitopes was strongly influenced by Tyr-overexpression providing evidence for possible active impairment of anti-vector immunity by modulation of epitopic dominance (Fig. 8 and 9).

In summary, we provide experimental evidence supporting the superior capacity of Prom Bor Prom C-based MVA vaccines to induce and to expand the strength and broadness of the cellular immune responses stimulated by Prom A-based constructs. It is expected that this knowledge will facilitate the development of more efficient prophylactic or therapeutic interventions against infectious or tumor diseases based on MVA vaccines. These findings have direct implications for the design of MVA vector vaccines suitable for induction of more potent T cell responses in experimental and clinical studies.

## Claims

1. A pharmaceutical composition comprising
a) a vaccinia virus (VV) based vector construct, wherein the vector construct encodes a heterologous antigen under the control of a strong VV specific promoter; and
b) a pharmaceutically acceptable carrier.

2. The composition of claim 1, wherein the heterologous antigen is a pathogenic antigen.

3. The composition of claim 2, wherein the pathogenic antigen is derived from the group consisting of viruses, bacteria, protozoa, fungi and parasites as well as tumor cells or tumor cell associated antigens and functional parts thereof.

4. The composition of claim 3, wherein the viruses are selected from the group consisting of influenza viruses, measles and respiratory syncytial viruses, dengue viruses, human immunodeficiency viruses, human hepatitis viruses, herpes viruses, or papilloma viruses.

5. The composition of claim 3, wherein the construct encodes an antigen selected from carcinoembryonic antigen (CEA), Her-2/neu, Gag of HIV and PA derived from the polymerase complex of Influenza.

6. The composition of one or more of the preceding claims, wherein the VV is modified vaccinia virus Ankara (MVA).

7. The composition of one or more of the preceding claims, wherein the promoter is a promoter having strong activity in the early and/or late phase of the viral life cycle.

8. The composition of claim 7, wherein the promoter is Prom B or Prom C.

9. The composition of claim 8, wherein the promoter has the sequence of SEQ ID NO:1 or 2.

10. The composition of one or more of the preceding claims, wherein the VV is MVA and the site of insertion of the sequence encoding the heterologous antigen is a non essential site in the viral genome, preferably deletion III.

11. A combined composition comprising the following ingredients:
a) a first composition according to one or more of the preceding claims, provided for priming;
b) a second composition according to one or more of the preceding claims, provided for boosting.

12. The pharmaceutical composition of one or more of the preceding claims, which is a vaccine.

13. Use of the pharmaceutical composition of claims 1-12 in the manufacture of a medicament for the treatment of infectious diseases, chronic inflammatory or degenerative diseases, autoimmunity diseases, fungal diseases, cancer and allergies.

14. Use of the pharmaceutical composition of claims 1-12 in evoking an immune response against subdominant and/or cryptic epitopes.

15. A method of evoking an immune response in a patient against an antigen, comprising the steps of:
a) providing a pharmaceutical composition of claims 1-12,
b) administering said composition to said patient in a suitable amount,
thereby evoking an immune response in said patient.

16. The method of claim 15, wherein the immune response is a cellular immune response.

17. The method of claims 15 and 16, wherein the patient is a mammalian, preferably a human patient.

18. The method of one or more of claims 15-17, comprising the following steps:
a) priming of a mammal with a therapeutically effective amount of a pharmaceutical composition of one or more of claims 1-12,
b) optionally repeating said step a) between one and three times after between one week and eight months; and
c) boosting of the mammal with a therapeutically effective amount of the same pharmaceutical composition used in a).

19. The method according to claim 18 wherein the priming steps are carried out twice prior to boosting.

20. The method according to claim 19 wherein the priming steps are carried out at the beginning of the treatment and in week three to five, preferably week four of the immunization, wherein the boosting step is carried out in week eleven to thirteen, preferably week twelve of the immunization.
